# EUROPEAN PATENT APPLICATION

(11) **EP 1 431 308 A1**
(43) Date of publication of application: **23.06.2004**
(21) Application number: 02777794.5
(22) Date of filing: 25.09.2002
(51) Int. Cl.: C07K 14/47, C12N 9/12, C07K 16/40

(54) **ENZYME PHOSPHORYLATING SER46 OF P53**

(30) Priority: 26.09.2001 JP 2001292953
(71) Applicant: Japan Science and Technology Agency, Kawaguchi-shi, Saitama 332-0012 (JP); JAPAN as represented by PRESIDENT OF NATIONAL CANCER CENTER, Chuo-ku, Tokyo 104-0045 (JP)
(72) Inventor: TAYA, Yoichi, Setagaya-ku, Tokyo 158-0083 (JP); TANAKA, Tomoaki, Chiba 286-0205 (JP); NAKAMURA, Yusuke, Yokohama-shi, Kanagawa 225-0011 (JP); ARAKAWA, Hirofumi, Suginami-ku, Tokyo 167-0035 (JP)
(74) Representative: Wilson Gunn Gee
(86) International application number: PCT/JP2002/009914
(87) International publication number: WO 2003/027144

(57) **Abstract**

The inventors clarified the enzyme phosphorylating the serine residue at amino acid position 46 of tumor suppressor protein p53 and further elucidated the mechanism in regulation of inducibility of apoptosis by p53. As the results, the inventors provide a completely new enzyme comprising casein kinase 2 and p53DINP1

SEQUENCE LISTING

<110> Japan Science and Technology Corporation

<120> The enzyme phosphorylating Ser46 of p53

<130> FS02-287PCT

<160> 5

<210> 1

<211> 391

<212> PRT

<213> casein kinase 2α

<400> 1
<210> 2
<211> 350
<212> PRT
<213> casein kinase 2α'
<400> 2
Met Pro Gly Pro Ala Ala Gly Ser Arg Ala Arg Val Tyr Ala Glu Val
1               5                   10                  15
Asn Ser Leu Arg Ser Arg Glu Tyr Trp Asp Tyr Glu Ala His Val Pro
            20                  25                  30
Ser Trp Gly Asn Gln Asp Asp Tyr Gln Leu Val Arg Lys Leu Gly Arg
        35                  40                  45
Gly Lys Tyr Ser Glu Val Phe Glu Ala Ile Asn Ile Thr Asn Asn Glu
    50                  55                  60
Arg Val Val Val Lys Ile Leu Lys Pro Val Lys Lys Lys Lys Ile Lys
65                  70                  75                  80
Arg Glu Val Lys Ile Leu Glu Asn Leu Arg Gly Gly Thr Asn Ile Ile
                85                  90                  95
Lys Leu Ile Asp Thr Val Lys Asp Pro Val Ser Lys Thr Pro Ala Leu

            100                 105                 110
Val Phe Glu Tyr Ile Asn Asn Thr Asp Phe Lys Gln Leu Tyr Gln Ile
        115                 120                 125
Leu Thr Asp Phe Asp Ile Arg Phe Tyr Met Tyr Glu Leu Leu Lys Ala
    130                 135                 140
Leu Asp Tyr Cys His Ser Lys Gly Ile Met His Arg Asp Val Lys Pro
145                 150                 155                 160
His Asn Val Met Ile Asp His Gln Gln Lys Lys Leu Arg Leu Ile Asp
                165                 170                 175
Trp Gly Leu Ala Glu Phe Tyr His Pro Ala Gln Glu Tyr Asn Val Arg
            180                 185                 190
Val Ala Ser Arg Tyr Phe Lys Gly Pro Glu Leu Leu Val Asp Tyr Gln
        195                 200                 205
Met Tyr Asp Tyr Ser Leu Asp Met Trp Ser Leu Gly Cys Met Leu Ala
    210                 215                 220
Ser Met Ile Phe Arg Arg Glu Pro Phe Phe His Gly Gln Asp Asn Tyr
225                 230                 235                 240
Asp Gln Leu Val Arg Ile Ala Lys Val Leu Gly Thr Glu Glu Leu Tyr
                245                 250                 255
Gly Tyr Leu Lys Lys Tyr His Ile Asp Leu Asp Pro His Phe Asn Asp
            260                 265                 270
Ile Leu Gly Gln His Ser Arg Lys Arg Trp Glu Asn Phe Ile His Ser
        275                 280                 285
Glu Asn Arg His Leu Val Ser Pro Glu Ala Leu Asp Leu Leu Asp Lys
    290                 295                 300
Leu Leu Arg Tyr Asp His Gln Gln Arg Leu Thr Ala Lys Glu Ala Met

305                 310                 315                 320
Glu His Pro Tyr Phe Tyr Pro Val Val Lys Glu Gln Ser Gln Pro Cys
                325                 330                 335
Ala Asp Asn Ala Val Leu Ser Ser Gly Leu Thr Ala Ala Arg
            340                 345                 350
<210> 3
<211> 215
<212> PRT
<213> casein kinase 2β
<400> 3
Met Ser Ser Ser Glu Glu Val Ser Trp Ile Ser Trp Phe Cys Gly Leu
  1               5                  10                  15
Arg Gly Asn Glu Phe Phe Cys Glu Val Asp Glu Asp Tyr Ile Gln Asp
             20                  25                  30
Lys Phe Asn Leu Thr Gly Leu Asn Glu Gln Val Pro His Tyr Arg Gln
         35                  40                  45
Ala Leu Asp Met Ile Leu Asp Leu Glu Pro Asp Glu Glu Leu Glu Asp
     50                  55                  60
Asn Pro Asn Gln Ser Asp Leu Ile Glu Gln Ala Ala Glu Met Leu Tyr
 65                  70                  75                  80
Gly Leu Ile His Ala Arg Tyr Ile Leu Thr Asn Arg Gly Ile Ala Gln
                 85                  90                  95
Met Leu Glu Lys Tyr Gln Gln Gly Asp Phe Gly Tyr Cys Pro Arg Val
            100                 105                 110
Tyr Cys Glu Asn Gln Pro Met Leu Pro Ile Gly Leu Ser Asp Ile Pro
        115                 120                 125

Gly Glu Ala Met Val Lys Leu Tyr Cys Pro Lys Cys Met Asp Val Tyr
    130                 135                 140
Thr Pro Lys Ser Ser Arg His His His Thr Asp Gly Ala Tyr Phe Gly
145                 150                 155                 160
Thr Gly Phe Pro His Met Leu Phe Met Val His Pro Glu Tyr Arg Pro
                165                 170                 175
Lys Arg Pro Ala Asn Gln Phe Val Pro Arg Leu Tyr Gly Phe Lys Ile
            180                 185                 190
His Pro Met Ala Tyr Gln Leu Gln Leu Gln Ala Ala Ser Asn Phe Lys
        195                 200                 205
Ser Pro Val Lys Thr Ile Arg
    210                 215
<210> 4
<211> 240
<212> PRT
<213> p53Dinp1a
<400> 4
Met Phe Gln Arg Leu Asn Lys Met Phe Val Gly Glu Val Ser Ser Ser
1               5                   10                  15
Ser Asn Gln Glu Pro Glu Phe Asn Glu Lys Glu Asp Asp Glu Trp Ile
            20                  25                  30
Leu Val Asp Phe Ile Asp Thr Cys Thr Gly Phe Ser Ala Glu Glu Glu
        35                  40                  45
Glu Glu Glu Glu Asp Ile Ser Glu Glu Ser Pro Thr Glu His Pro Ser
    50                  55                  60
Val Phe Ser Cys Leu Pro Ala Ser Leu Glu Cys Leu Ala Asp Thr Ser

65                  70                  75                  80

Asp Ser Cys Phe Leu Gln Phe Glu Ser Cys Pro Met Glu Glu Ser Trp

                85                  90                  95

Phe Ile Thr Pro Pro Pro Cys Phe Thr Ala Gly Gly Leu Thr Thr Ile

            100                 105                 110

Lys Val Glu Thr Ser Pro Met Glu Asn Leu Leu Ile Glu His Pro Ser

        115                 120                 125

Met Ser Val Tyr Ala Val His Asn Ser Cys Pro Gly Leu Ser Glu Ala

    130                 135                 140

Thr Arg Gly Thr Asp Glu Leu His Ser Pro Ser Ser Pro Arg Val Glu

145                 150                 155                 160

Ala Gln Asn Glu Met Gly Gln His Ile His Cys Tyr Val Ala Ala Leu

                165                 170                 175

Ala Ala His Thr Thr Phe Leu Glu Gln Pro Lys Ser Phe Arg Pro Ser

            180                 185                 190

Gln Trp Ile Lys Glu His Ser Glu Arg Gln Pro Leu Asn Arg Asn Ser

        195                 200                 205

Leu Arg Arg Gln Asn Leu Thr Arg Asp Cys His Pro Arg Gln Val Lys

    210                 215                 220

His Asn Gly Trp Val Val His Gln Pro Cys Pro Arg Gln Tyr Asn Tyr

225                 230                 235                 240

<210> 5

<211> 164

<212> PRT

<213> p53Dinp1b

<400> 5

Met Phe Gln Arg Leu Asn Lys Met Phe Val Gly Glu Val Ser Ser Ser
1               5                   10                  15
Ser Asn Gln Glu Pro Glu Phe Asn Glu Lys Glu Asp Asp Glu Trp Ile
            20                  25                  30
Leu Val Asp Phe Ile Asp Thr Cys Thr Gly Phe Ser Ala Glu Glu Glu
        35                  40                  45
Glu Glu Glu Glu Asp Ile Ser Glu Glu Ser Pro Thr Glu His Pro Ser
    50                  55                  60
Val Phe Ser Cys Leu Pro Ala Ser Leu Glu Cys Leu Ala Asp Thr Ser
65                  70                  75                  80
Asp Ser Cys Phe Leu Gln Phe Glu Ser Cys Pro Met Glu Glu Ser Trp
                85                  90                  95
Phe Ile Thr Pro Pro Pro Cys Phe Thr Ala Gly Gly Leu Thr Thr Ile
            100                 105                 110
Lys Val Glu Thr Ser Pro Met Glu Asn Leu Leu Ile Glu His Pro Ser
        115                 120                 125
Met Ser Val Tyr Ala Val His Asn Ser Cys Pro Gly Leu Ser Glu Ala
    130                 135                 140
Thr Arg Gly Thr Asp Glu Leu His Ser Pro Ser Ser Pro Arg Ala Arg
145                 150                 155                 160
Lys Ser Cys Leu

## Description

### Technical field:

This invention relates to the enzyme which phosphorylate the serine residue at amino acid position 46 (hereinafter called "Ser46") of tumor suppressor protein p53.

### Prior art:

p53 induces G1 arrest in some cases, whereas it induces apoptosis in other cases. The mechanism how these two different processes are selected has been important to elucidate but remained unexplained. The inventors found, for the first time in the world, that newly phosphorylated Ser46 of p53 regulates apoptosis (Oda, K. et al., Cell, 102, 849-862, 2000).

The above story is based on our hypothesis advocated three years ago. The hypothesis is such as in the followings: A kind of DNA damage may induce phosphorylation of a certain position of p53, which could result in binding of p53 to promoter region of the genes involved in G1 arrest, such as p21^{Waf1}. Such promoter-p53 binding should induce expression of the genes to cause G1 arrest. In contrast, another kind of DNA damage may induce phosphorylation of other parts of p53, which could result in binding of p53 to promoter region of genes involved in apoptosis. Such promoter-p53 binding must induce expression of the apoptosis-related genes and finally apoptosis.

The inventors also inferred which region of p53 may regulate inducibility of apoptosis (Experimental Medicine, 18: 2212-2218, 2000). Two domains of p53 were expected to be involved in the region. One is proline-rich domain (amino acid 64 to 101), closed toN-terminal region of the DNA binding domain. The other is a domain consisting of only 20 amino acids (amino acid 43 to 63) , which is located outside the N-terminal region of the proline-rich domain.

Comparing the amino acid sequence of these two domains, the inventors noticed three possible *in vivo* phosphorylation sites (Ser46, thr81 and Ser90). Probably Ser46 might be phosphorylated by cycline-dependent kinase or MAP kinase, since the next amino acid to Ser46 is Pro.

Then, the inventors started experiments to verify our prediction using antibodies, which recognize specifically these phosphorylated sites. In the beginning, the inventors found that the phopholylation of Ser46 always took place at later time points than that of Ser15 or Ser20, after cells were exposed to γ irradiation, UV light or adriamycin to induce DNA damage. Furthermore, the induction of phosphorylation of Ser46 needs stronger intensity of UV light than that for Ser15 or Ser20, when cells were exposed to UV with changed intensity. Therefore, the inventors thought that the phosphorylation may coincide with apoptosis.

Last July, the inventors noticed the late expression of p53AIP1 (p53 regulated apoptosis inducing protein 1), compared with p21^{waf1}, and the similarity between the expression of p53AIP1 and Ser46 phosphorylation. Then, the inventors started new research.

The research gave us much interesting development unexpectedly. Changing Ser46 to Ala resulted in marked reduction in inducibility of apoptosis by p53. Furthermore, among many target genes of p53, only expression of p53AIP1 was found to be effectively influenced by Ser46 substitution. On the contrary, there are no influence of Ser46 substitution found on Bax, PIG3 or NOXA, which are suggested by other groups to be involved in apoptosis.

According to our literature review after getting the above results, the inventors found some reports on mutation of Ser46 and Pro47 to other amino acids in some cases for human cancer. The inventors felt that the authors had reported the results without understanding the meaning. To the inventor's interpretation based on the test result, these human cases may show mutations lacking in phosphorylation of Ser46.

Based on these results, the inventors constructed the following model (Experimental Medicine, 18: 2338-9, 2000): DNA damage might induce the phosphorylation of Ser15 and Ser20, leading to the stabilization and activation of p53, and p53 may bind to the promoters of the genes related to G1 arrest, such as p21^{waf1} or the genes related to DNA repair, such as p53R2, to induce these expression. However, severe DNA damage could not be recovered by G1 arrest and DNA repair, instead, could induce activation of Ser46-kinase and phosphorylation of Ser46 of p53, which increased affinity to promoter region of p53AIPI gene, and accordingly activate expression of p53AIP1, which leads to apoptotic cell death.

Recently, it has been suggested that p53DINP1 (p53 damage inducible nuclear protein 1) could be a constituent of the enzyme phosphorylating Ser46 of p53 (i.e., Ser46-kinase) (Molecular Cell, 8, 85-94, 2001), since not only the expression of p53DINP1 but also phosphorylation of Ser46 are repressed when cells are treated with antisense oligonucleotides corresponding to the p53DINP1 gene.

Current radiation therapy or chemotherapy by adriamycin, for example, could be interpreted as inducing apoptotic death of tumor cells, by damaging DNA and activating this pathway. Therefore, DNA in normal cells could also be damaged and side effects might be emerged. However, the mechanism, which the inventors elucidated, could be expected to apply to a development of a new therapy, in which only tumor cells could be induced to apoptotic death without damaging DNA.

### Problems to be resolved by the invention:

The invention is to provide a completely new enzyme, obtained during the research on the enzymatic phosphorylation of the serine residue at amino acid position 46 of tumor suppressor protein p53 and further on the elucidation of the mechanism in regulation of inducibility of apoptosis by p53.

### Means to solve the problems and detailed description of the invention:

The inventors succeeded for the first time in isolation of the enzyme, which phosphorylate Ser46 and elucidated their characteristics: The inventors clarified that the enzyme contains casein kinase 2 (hereinafter also called "CK2") and that the enzyme is a protein complex of CK2 and p53DINP1 (Molecular Cell, 8, 85-94, 2001).

Therefore, the invention is an enzyme comprising casein kinase 2 and p53DINP1. CK2 is a commonly known kinase, being an ubiquitous serine / threonine kinase present in eukaryotic cells. CK2 is composed of α or α' subunit and β subunit in the tetrameric form (i.e. α *α β β* or *α α'* β β) and is involved in the regulation of cell cycle and various signal transduction, phosphorylating various substrates, such as oncoproteins, transcription factors and enzymes related to DNA metabolism (Litchfield, D.W., et al. Mol. Cell. Biochem. 1993 Vol127, :p187-199, Allende, J.E. et al., FASEB J. 1995, Vol9:p313-323) . On the other hand, p53DINP1 is one of the commonly known target proteins of p53, identified in 2001 and is a nuclear protein (there exist two types, a and b), inducible by DNA damage. P53DINP1 plays an important role in p53-dependent apoptosis, but the molecular mechanism remained unexplained (S. Okamura, et al. Molecular Cell 2001 Vol8: p85-94). The relevant enzyme is a protein complex of CK2 and P53DINP1 and phosphorylates Ser46 of p53.

The size of this enzyme is about 250 - 350kDa, as described in later examples. Whereas, the size of CK2 is about 130 - 144kDa (α subunit is about 44kDa; α' subunit is about 37 kDa; β subunit is about 28kDa) and that of p53DINP1a and b are about 42kDa and 38 kDa, respectively. Therefore, although the enzyme is a protein complex of CK2 and P53DINP1, the protein complex contains other factors than CK2 and p53DINP1.

The invention is an enzyme comprising at least one selected from the group consisting of proteins having one of the amino acid sequences of sequence numbers 1 to 3 and also at least one selected from the group consisting of proteins having one of the amino acid sequences of sequence numbers 4 and 5. The proteins having the amino acid sequences of sequence numbers 1 to 5 correspond to α, α' and β subunits of CK2, and p53DINPla and b, respectively.

Viewed from a different standpoint, this invention is an enzyme, which is a nuclear fraction obtained during purification of cells with activated p53, said nuclear fraction reacting with an antibody specific to an antigen that comprises a peptide fragment of p53 containing Ser46, said Ser46 being phosphorylated, when p53 being added to said nuclear fraction, and reacts with an antibody specific to casein kinase 2. The invention is also the enzyme, which further reacts with an antibody specific to p53DINP1.

The nuclear fractions may be recovered from objective cells by appropriate known method.

As for p53 to be added to the nuclear fractions, p53 recombinant protein purified in *E. coli.* is preferred.

Antibodies specific to CK2 or p53DINP1 may be prepared using known methods or obtained as commercial products.

As for whether the nuclear fractions contain activated p53, it is judged depending on whether the nuclear fraction may react with an antibody specific to an antigen that comprises a peptide fragment of p53 containing Ser46, said Ser46 being phosphorylated. This peptide fragment may comprise at least 4 to 5 amino acids containing Ser46 wherein this Ser46 is phosphorylated. The peptide fragment may generally be used by coupling with carrier proteins, such as Keyhole Limpet Hemocyanine (KLH) , which is separated about 5 residues from the Ser residue.

Exposure of target cells to UV irradiation, γ ray irradiation, oxidative stress of various anti-tumor drugs such as *cis*-Platinum, etoposide or adramycin, but not to osmotic shock, induces DNA damage and activates p53.

The isolation or purification of the enzyme could be done by any known methods. It is preferable to use at least one process of the column separation processes such as hydroxyapatite column separation, gel-filtration column separation or anion-exchange column separation, and further to use the process of either cation-exchange column purification or hydrophorbic column purification. It is preferred to combine at least 2 or 3 different processes among these isolation or purification processes

Injection, in some way, of the enzyme of this invention into tumor cells could induce apoptosis of the cell and lead to cell death. The drugs activating specifically the kinase activity of the enzyme, if obtained by screening, could be useful as anti-tumor agent. The drugs inhibiting specifically the activity of the enzyme, if obtained by screening, could be useful as alleviating drugs for side effects of chemotherapy using anti-tumor agents. Therefore, the invention also provides an anti-tumor agent comprising one of these enzymes as an effective component, a screening agents for drugs activating specifically p53, comprising one of these enzymes as an effective component, and a screening agents for drugs inhibiting one of the enzymes, comprising said enzyme as an effective component. The enzyme of this invention can be formulated by known methods to form these drugs. The inventions are also use of any of the enzymes as an anti-tumor agent, use of any of the enzymes as a screening agents for drugs activating specifically p53, and use of any of the enzymes as a screening agents for drugs inhibiting the activity of said enzyme.

### Brief explanation of the drawings

Figure 1 shows Western blotting as detected by anti-p53-phosphorylated Ser46 antibodies for the enzyme reaction mixture using the nuclear extracts with damaged DNA.
Figure 2 shows Western blotting as detected by anti-p53-phosphorylated Ser46 antibodies for enzyme reaction mixture using purified enzyme through Mono Q column after purified the nuclear extracts with damaged DNA through Superose 6 column.
Figure 3 shows *In gel* assay for the purified nuclear extracts with damaged DNA.
Figure 4 shows a silver stained SDS-PAGE gel for the nuclear extracts with damaged DNA.
Figure 5 shows Western blotting as probed by anti-p53-phosphorylated Ser46 antibodies for the enzyme reaction mixture obtained by immunoprecipitation of purified nuclear extracts (active fraction for phosphorylation of Ser46) by anti-CK2α antibodies.
Figure 6 shows Western blotting as probed by anti-p53-phosphorylated Ser46 antibodies for enzyme reaction mixture obtained by immunoprecipitation of purified nuclear extracts by anti-p53-phosphorylated Ser46 antibodies.
Figure 7 shows Western blotting as probed by anti-CK2 α antibodies (A), by anti-CK2 β antibodies (B) and by anti-p53DINP1 antibodies (C) for immune complex of nuclear extracts precipitated by anti-p53DINP1 antibodies (IgG), by anti-CK2 α antibodies and by anti-CK2 β antibodies, respectively.

### Effects of the invention

This invention is one of the results obtained during the research by the inventors, which successfully revealed that the enzyme phosphorylating the serine residue at amino acid position 46 of p53 is the enzyme comprising CK2 and p53DINP1 and clarified the regulatory mechanism in induction of apoptosis by p53. As a result, this invention provides a new enzyme. This new enzyme was purified through a series of difficult purification processes and its structure was determined.

The enzyme of this invention can be applied to the development of new therapeutic methods for cancer by introducing selective apoptotic death of tumor cells. Furthermore, the enzyme can be applied for screening of agents activating specifically kinase activity of the enzyme. Such activating agents can be used as new anti-tumor drugs. Furthermore, the enzyme can be applied for screening of agents inhibiting specifically kinase activity of the enzyme. Such inhibiting agents can be used as new alleviating drugs for chemotherapy using anti-tumor drugs.

### Examples

In the following paragraph, the inventors illustrate the practical examples of this invention, but do not intend to restrict the scope of the invention.

### (1) In the beginning, the phosphorylating activity of the enzyme induced by DNA damage was examined.

Human breast cancer cells MCF-7 (hereinafter called "MCF-7", ATCC) were cultured in DMEM medium (Sigma) containing 10 % fetal bovine serum (GIBCO BRL) on 15 cm² dishes (Corning) up to 90 % confluent state. These cells were treated with final 3 µM doxorubicin (Sigma, the same to adriamycin) by addition of its aqueous solution to the medium. After allowed to stand in an incubator, the cells were harvested at various time points.

The harvested cells from one dish were suspended and solubilized in 500 µl of cytoplasm extraction buffer (10mM HEPES pH7.9, 2mM MgCl₂, 0.1mM EDTA, 0.1mM EGTA, 1% Nonidet NP-40, DTT 1mM, Na₃VO₄ 1mM, NaF 5mM, PMSF 0.5mM, KCl 10mM, leupeptin 5 *µ*g/ml, antipain 5 *µ*g/ml, chymostatin 5 *µ*g/ml, pepstatinA 2 *µ*g/ml). The solbilized cells were centrifuged at 15, 000 rpm for 15min. The pellets obtained were mixed with 100 *µ*l nuclear extraction buffer (10mM HEPES pH7. 9, 2mM MgCl₂, 0.1mM EDTA, 0.1mM EGTA, 1% Nonidet NP-40, DTT 1mM, Na₃VO₄ 1mM, NaF 5mM, PMSF 0.5mM, KCl 10mM, NaCl 500mM, leupeptin 5*µ*g/ml, antipain 5 *µ*g/ml, chymostatin 5 *µ*g/ml, pepstatinA 2*µ*g/ml), suspended well and centrifuged at 15,000 rpm for 15 min. The supernatant obtained is adjusted to 10 mg protein/ ml buffer and is called as the nuclear extracts.

On the other hand, the inventors prepared the following polypeptides containing a phosphorylated serine residue at amino acid position 46 of p53 by a polypeptide synthesizer:

MDDLMS(PO₃H₂)PDDIEQC (sequence number 6)

In the sequence, S(PO₃H₂) indicates a phosphorylated serine residue.
The phosphorylated peptides were coupled covalently to KLH and used as antigens. Immunization was carried out by injecting these antigens together with adjuvant under a rabbit back skin. After five weeks, venous blood was collected from the rabbit earlobe. The antiserum was purified by two steps affinity chromatography and is called as anti-p53-phosphorylated-Ser46 antibody (Oda, K et al., Cell, 102,849-862, 2000).

Next, the enzyme activity of the nuclear extracts was assayed using the antibodies. As a substrate for the enzyme reaction, a fusion protein (GST-p53) between the amino acid of the whole p53 and glutathione-s-transferase (GST) was used. The reaction mixture containing 10 *µ*g of nuclear extracts and 1 *µ*g of GST-p53 in 40 *µ*l of kinase reaction buffer (25mM Tris-HCl (pH 7.5), 5 mM β -glycerophosphate, 2mM DTT, 0.1 mM Na₃VO₄, 10mMMgCl₂, 5mMATP) was kept at 30 °C for 30 min. After addition of SDS sample buffer (125mM Tris-HCl (pH 6.5) , 45% Glycerol, 5 %SDS, 5% β-mercaptoethanol, 0.125% BPB), the reaction mixture was heated at 100 °C for 5 min and was separated on a 10 % SDS-PEGE gel. The phosphorylation of GST-p53 was detected in Western blotting probed with anti-p53-phosphorylated-Ser46 antibodies.

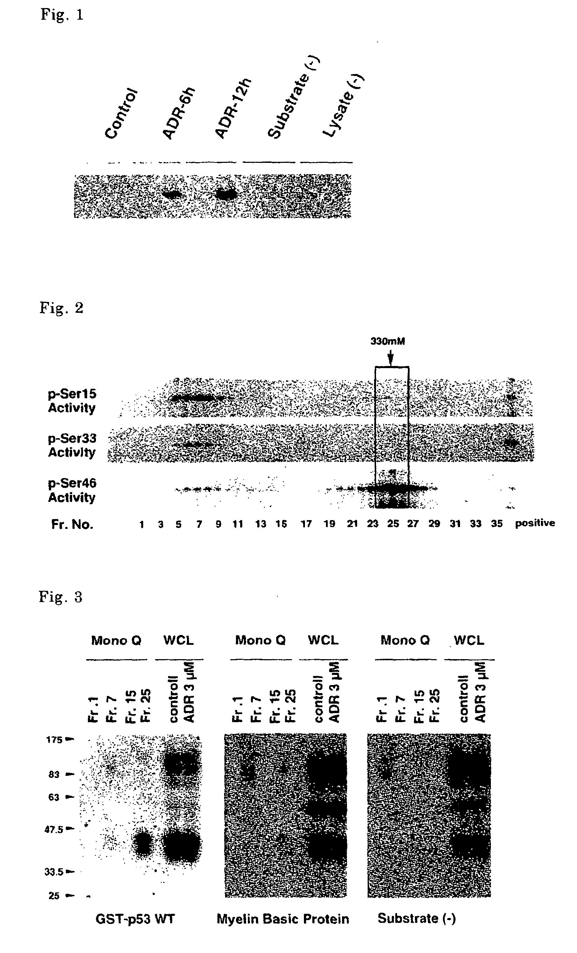

The results were shown in Fig.1. In this figure, ADR-6h shows the reaction between the nuclear extracts, isolated at 6 hours after doxorubicin treatment, and the substrate GTS-p53. ADR-12h shows that between the nuclear extracts, isolated at 12 hours after the doxorubicin treatment, and the substrate GTS-p53. Substrate (-) shows the reaction without the substrate and Lysate (-) shows the reaction without the nuclear extracts. It can be understood that only the reaction between the nuclear extracts, isolated after the doxorubicin treatment, and the substrate GST-p53 shows the presence of enzyme activity phosphorylating Ser46.

### (2) Then, the enzyme phosphorylating Ser46 of p53 was purified. As shown in (1), activation of enzyme phosphorylating Ser46 of p53 was greatly induced in cells during 12 hours after the treatment with doxorubicin. Purification of the enzyme phosphorylating Ser4 6 of p53 was carried out using the nuclear extracts as starting materials for column chromatography.

AKTA explorer chromatography system (Amersham-Pharmacia) was used for purification by FPLC. Connecting Superose 6 HR (Amersham-Pharmacia) to the system, the inventors eluted 5 mg of the nuclear extracts by the elution buffer (50 mM Tris pH 7.4, 1 mMEDTA, 2.5m MEGTA, 150mM Nacl, 0.1mM Sodium vanadate, 5%Glyecerol) at a flow rate of 0.2 ml/min, collecting 0.5 ml for a fraction. The activity to phosphorylate Se46 of p53 was measured for each fraction and the active fractions were used for the next purification.

Then, connecting MonoQ HR5/5 (Amasham-Pharmacia) to the system, equilibrating the column with starting buffer (50 mM Tris pH 7.4, 1 mM EDTA, 2.5 mM EGTA, 250 mM NaCl, 0.1 mM Sodium vanadate, 5 % Glyecerol) the inventors eluted the 500 µg of the active fractions by the elution buffer (50 mM Tris pH 7.4, 1 mM EDTA, 2.5 mM EGTA, 500 mM NaCl, 0.1 mM Sodium vanadate, 5 % Glyecerol) at a flow rate of 0.3 ml/ min, collecting 0.5ml for a fraction by gradient elution. The enzyme activity for each fraction was shown in Fig. 2 (Fig. 2, p-Ser46 Activity). As a result, a peak of the activity can be seen at fr. 25, where the salt concentration is around 330 mM. In Fig. 2, p-Ser15 Activity and p-Ser33 Activity were the results obtained using a similar procedure to p-Ser46 Activity using anti-phosphorylated Ser15 antibodies and anti-phosphorylated Ser33 antibodies, respectively, for comparison.

### (3) Then, the purified enzyme was identified.

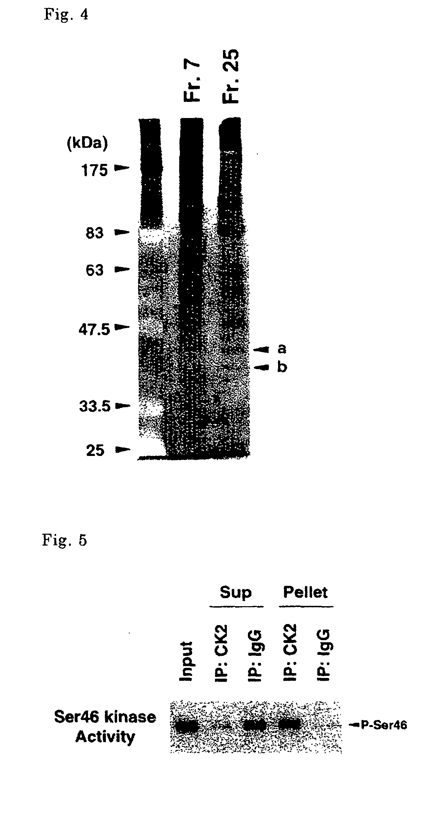

In the beginning, the molecules responsible for the activity phosphorylating Ser46 was identified by *In Gel* assay. Several fractions (1, 7, 15 and 25) from anion exchange column chromatography (MonoQ) as shown in Fig. 2 (2) were separated by electrophoresis through SDS-PAGE gels containing GST-p53 and Myelin basic proteins, respectively, as substrates for kinase activity. After the electrophoresis, SDS was removed from the gels. The kinase activity of the separated proteins was detected by autoradiography after In Gel assay in the presence of ³²P-ATP. The results are shown in Fig. 3. As shown in Fig. 3, using GST-p53 as substrates, strong p53 phosphorylating activity was detected in the fraction 25, in which the enzyme phosphorylating Ser46 of p53 was detected using anti-p53-phosphorylated Ser46 antibodies. However, kinase activity was not detected without using substrates (substrate (-) ) or with Myelin basic protein, not containing p53, as substrates. It can be understood that the enzyme induced after DNA damage shows the phosphorylating activity only when the enzyme encounters p53.

Gel filtration chromatography of the enzyme shows that the molecular weight of the purified enzyme is around 250 - 350 Kda.

Precise examination of the active portion of the enzyme revealed that the portion is consists of two parts. Figure 4 shows silver staining of the enzyme phosphorylating Ser46 in purified fraction (shown as "a" and "b" in Fig. 4).

Fragments a and b were cut out from SDS-PAGE gel, were washed two times in 25 mM Sodium bicarbonate/ 50% acetonitril, were added Tris-buffer (pH8.0) containing trypsin, were digested at 35 C for 20 hrs and the 70% portion of the digested samples were analyzed by LC-MS/MS (The analytical condition. HPLC apparatus: MAGIC 2002 (Michrom BioResources, Inc., USA) Column : MAGIC 2002 (Michrom BioResources, Inc., USA); Solvent A: 2% acetnitril/ 0.1% formic acid; Solvent B: 90% anetnitril/ 0.1% formic acid; Flow rate: 400~500 1/min; Gradient: 0 min, 5 (B%), 25 min, 65 (B%), 26 min, 100 (B%), 27 min, 100 (B%), 28 min, 5 (B%); Mass sectrometer: Q-Tof2 (Micromass, UK); Ionaization method: Nanoflow-LC ES1; Ionization mode: Positive mode, Capillary voltage: 2 Kv; Collision energy: 20-35 eV.) The amino acid sequence of the Ser46 phosphorylating enzyme was determined by this LC-MS/MS analysis (The part a corresponds to the sequence number 1; the part b corresponds to the sequence number 2) and fragments a and b in Fig. 4 were proved as α and α' subunits of CK2, respectively.

### (4) Then, it was confirmed that CK2 is included in the purified enzyme by immunoprecipitation method.

The fraction 25 described in (2) was immunoprecipitated with anti-CK2α antibody (Santacruse). The supernatant and the immune complexes were assayed their Ser46 phosphorylating activity and the results are shown in Fig. 5A. Figure 5A shows that the activity was disappeared in the supernatant and was transferred to the pellet, which includes CK2. Immunoprecipitation by non-specific antibodies (IgG) does not show any Ser46 phosphorylating activity.

### (5) Then, it was confirmed that p53DINP1 is included in the purified Ser46 phosphorylating enzyme by immunoprecipitation method.

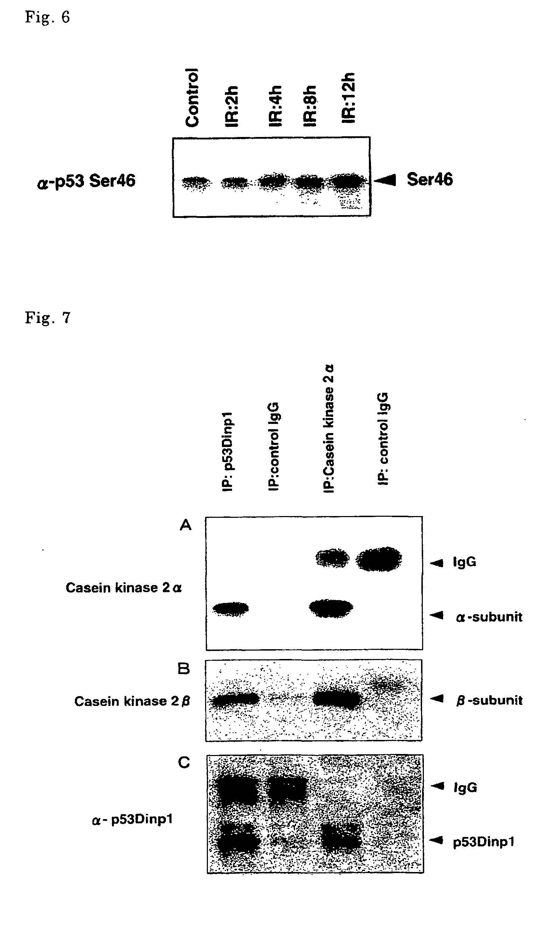

MCF-7 cells were harvested at various time points after the exposure to 30 Gy of γ ray and the cell lysates were prepared. Each sample was immunoprecipitated with anti-p53 DINP1 antibodies. Kinase assay was performed using the immunoprecipitation as the enzyme source and GST-p53 as substrates. The immune complex was separated by SDS-PAGE and the Western blotting using anti-p53-phosphorylating antibodies are shown in Fig. 6. It shows that the phospholulating enzyme includes p53DINP1.

The anti-p53PDINP1 antibodies were prepared as follows:p53DIPN1a recombinant protein adding His residue at the N-terminal was expressed in *E. coli.* The protein was purified using a Ni-chelating sepharose column (Amersham-Pharmacia). Polyclonal, anti-p53DINP1 antibodies were prepared by injecting the purified protein as antigens together with adjuvants into rabbit.

### (6) Here, it was confirmed that CK2 and p53DINP1 are constructing a complex in vivo.

Twelve hours after the treatment of MCF-7 cells with adiramycin, cells were harvested, were suspended in 500 *µ*l of immunoprecipitation buffer (50 mM Hepes pH 7.4, 150 mM NaCl, 1 mM EDTA, 2.5 mM EGTA, 0.1 % Tween 20, 10 % glycerol, 1 mM NaF, 0.1 mM Sodium Vanadate, 0.5 mM PMSF, 5 *µ*g/ml Leupeptin, 5 *µ*g/ml chymostatin, 2 µg/ml pepstatin) per a dish cells in ice box and were crushed by sonication. The supernatant of the crushed cells by centrifugation is called as cell extracts after adjusting the concentration as final 10 mg proteins/ ml. Each 1 mg of the cell extracts was added 5 µg each of primary antibodies (anti-p53DINP1 antibody, anti-CK2α antibodies or nonspecific antibodies (IgG)). The mixtures were rotated slowly at 4 °C for 1 hr. Then, the mixtures were rotated slowly at 4 °C for 1 hr after adding 20 µg of proteinG-sepharose 4B (Amersham-Pharmacia). The pellets of the mixture after centrifugation were washed four times with the immunoprecipitation buffer. Then, the immune complex was eluted from the pellets by the sample buffer (125 mM Trs-HCl (pH 6.5), 45 % Glycerol, 5 % SDS, 5 % β-mercaptoethanol, 0.125 % BPB) after heating at 100 °C for 5 min. The obtained immune complex was separated by SDS-PAGE and the Western blotting probed with anti-p53DINP1 antibodies, anti-CK2 α antibodies or nonspecific antibodies (IgG) are shown in Fig. 7. Figure 7 shows that all of the antibodies as shown for anti-CK2 α antibodies (Fig. 7 A), anti-CK2 β antibodies (Fig. 7B) or anti-p53DINP1 antibodies (Fig. 7C) reacted with the immune complex precipitated both by anti-p53DINP1 antibodies and anti-CK2 α antibodies in Western blotting. This result means that CK2α, CK2 β and p53DINPl are composing a protein complex.

From the above embodiments, it was elucidated that the enzyme phosphorylating Ser46 of p53 is a protein complex composed of CK2 and p53DINP1.

## Claims

1. An enzyme comprising casein kinase 2 and p53DINP1.

2. An enzyme comprising at least one selected from the group consisting of proteins having one of the amino acid sequences of sequence numbers 1 to 3 and also at least one selected from the group consisting of proteins having one of the amino acid sequences of sequence numbers 4 and 5.

3. The enzyme as in claim 2, wherein one or a few amino acid(s) is/are deleted, substituted or added in said amino acid sequences of sequence numbers 1 to 5.

4. An enzyme, which is a nuclear fraction obtained during purification of cells with activated p53, said nuclear fraction reacting with an antibody specific to an antigen that comprises a peptide fragment of p53 containing Ser46, said Ser46 being phosphorylated, when p53 being added to said nuclear fraction, and reacts with an antibody specific to casein kinase 2.

5. The enzyme as in claim 4, which further reacts with an antibody specific to p53DINP1.

6. An anti-tumor agent comprising the enzyme as in any one of claims 1 to 5 as an effective component.

7. A screening agents for drugs activating specifically p53, comprising the enzyme as in any one of claims 1 to 5 as an effective component.

8. A screening agents for drugs inhibiting the enzyme as in any one of claims 1 to 5, comprising said enzyme as an effective component.

9. Use of the enzyme as in any one of claims 1 to 5 as an anti-tumor agent.

10. Use of the enzyme as in any one of claims 1 to 5 as a screening agents for drugs activating specifically p53.

11. Use of the enzyme as in any one of claims 1 to 5 as a screening agents for drugs inhibiting the activity of said enzyme.
